# EUROPÄISCHE PATENTSCHRIFT

(11) **EP 2 138 182 B1**
(45) Veröffentlichungstag und Bekanntmachung des Hinweises auf die Patenterteilung: **26.08.2015**
(21) Anmeldenummer: 09163367.7
(22) Anmeldetag: 22.06.2009
(51) Int. Cl.: A61K 36/232, A61K 36/23, A61K 36/484, A61K 36/28, A61K 36/31, A61K 36/53, A61K 36/534, A61K 36/66, A61P 1/00

(54) **Verfahren zur Herstellung eines Arzneimittels auf pflanzlicher Basis**
Method for producing medicine based on herbs
Procédé de fabrication d'un médicament à base végétale

(30) Priorität: 26.06.2008 DE 102008002685
(43) Veröffentlichungstag der Anmeldung: 30.12.2009
(73) Patentinhaber: Steigerwald Arzneimittelwerk GmbH, 64295 Darmstadt (DE)
(72) Erfinder: Kuper, Willi, 68649, Groß-Rohrheim (DE); Becker, Dr. Wulf, 64372, Ober-Ramstadt (DE)
(74) Vertreter: Wablat, Wolfgang

(56) Entgegenhaltungen:
- WO-A1-93/02688
- KROLL U ET AL: "Pharmaceutical prerequisites for a multi-target therapy", PHYTOMEDICINE, GUSTAV FISCHER VERLAG, STUTTGART, DE, Bd. 13, 24. November 2006 (2006-11-24), Seiten 12-19, XP025162795, ISSN: 0944-7113, DOI: DOI:10.1016/J.PHYMED.2006.03.016 [gefunden am 2006-11-24]

## Beschreibung

Die vorliegende Erfindung betrifft ein Verfahren zur Herstellung eines Arzneimittels auf pflanzlicher Basis, welches Iberis amara, Menthae piperitae folium, Matricariae flos, Carvi fructus, Melissae folium, Angelicae radix, Liquiritiae radix, Cardui mariae fructus und Chelidonii herba in Form alkoholischer Extrakte enthält und ein nach diesem Verfahren hergestelltes Arzneimittel sowie dessen Verwendung.

lberogast ist ein Arzneimittel auf pflanzlicher Basis (pflanzliches Kombinationspräparat, Phytopharmakon), welches zur Behandlung der funktionellen Dyspepsie und bei der gastrointestinalen Multitarget-Therapie eingesetzt wird. Es wirkt sowohl tonuserhöhend bei erschlaffter glatter Muskulatur als auch spasmolytisch bei krampfartigen Zuständen der glatten Muskulatur. Die arzneilich wirksamen Bestandteile dieses Phytopharmakons sind Zubereitungen aus Frischpflanzen und pflanzlichen Drogen, (getrocknete Pflanzen oder Pflanzenteile) als ethanolische, flüssige Extrakte. Iberogast enthält Extrakte aus Iberis amara (bittere Schleifenblume), Menthae piperitae folium (Pfefferminzblätter), Matricariae flos (Kamillenblüten), Carvi fructus (Kümmelfrüchte), Melissae folium (Melissenblätter), Angelicae radix (Angelicawurzel), Liquiritiae radix (Süßholzwurzel), Cardui mariae fructus (Mariendistelfrüchte) und Chelidonii herba (Schöllkraut).

Das Europäische Patent EP 0550 703 B1 beschreibt bereits ein Arzneimittel auf pflanzlicher Basis mit begrenzter Anzahl von 6 Extrakten (Auszügen).

Iberogast selbst, welches 9 Extrakte umfasst, ist ein seit Jahren gut etabliertes Arzneimittel, welches in der Literatur u.a. von Kroll et al. beschrieben wird (Kroll et al., Phytomedicine, 2006, 13, Seiten 12-19). Allerdings gibt auch diese Publikation keine definierte Mischungsreihenfolge der einzelnen Komponenten an. Es wird lediglich eine willkürliche Reihenfolge der einzelnen Bestandteile angeführt. Pauschal wird lediglich darauf hingewiesen, dass die richtige Mischungsreihenfolge wichtig ist. Weitere Indikatoren hinsichtlich der Reihenfolge werden nicht gegeben, es werden nur mehrere, möglicherweise relevante Faktoren wie beispielsweise Mischdauer, Lagerzeit und Filtration erwähnt.

Die pharmazeutische Qualität der Extrakte hat entscheidenden Einfluss auf die Wirksamkeit und Unbedenklichkeit pflanzlicher Arzneimittel. Neben der Einhaltung von arzneibuchkonformen Spezifikationen der Ausgangsdrogen ist die Reproduzierbarkeit der Herstellungsprozesse (Validierung) die wesentliche Voraussetzung für den hohen Standard als deutsches und europäisches Phytopharmakon. Basierend auf den neuen Leitlinien, welche auch für pflanzliche Arzneimittel im Bezug zu den betreffenden europäischen Regularien gelten, besteht die Verpflichtung, den für das jeweilige Arzneimittel deklarierten Angaben zum Gehalt zu entsprechen. Die Abweichung von diesen Angaben darf nur ± 5 % des deklarierten Gehaltes der wirksamkeitsbestimmenden Inhaltsstoffe (Leitsubstanz, aktiver Marker oder analytische Marker-Substanz) betragen.

Es hat sich als problematisch erwiesen, einen konstanten Wirkstoffgehalt im Fertigarzneimittel Iberogast und eine gleich bleibende, reproduzierbare Qualität zu erhalten. Entsprechende Untersuchungen zur pharmazeutischen Entwicklung deuteten auf Fällungen/Kristallbildung im Auszug aus Angelikawurzel hin, welche zu Inhomogenitäten im Produktionsansatz und in der Folge zu nicht akzeptablen Schwankungen des Osthol-Gehalts (Leitsubstanz) führten. Einerseits stieg die Wiederfindungsrate des Ostholgehaltes in den ersten Wochen nach der ersten Analyse in klarfiltrierten Lösungen an und andererseits sank der Gesamtgehalt in Produktionsansätzen entsprechend der Sedimentbildung.

Daher war es Aufgabe der vorliegenden Erfindung, einen konstanten Wirkstoffgehalt im Fertigarzneimittel mit einer gleich bleibenden, reproduzierbaren Qualität in der Endverpackung über einen längeren Zeitraum zu ermöglichen.

Diese Aufgabe wurde durch ein Verfahren zur Herstellung eines Arzneimittels auf pflanzlicher Basis gemäß Anspruch 1 gelöst. In anderen Worten wird diese Aufgabe durch ein Verfahren zur Herstellung eines Arzneimittels auf pflanzlicher Basis, welches Iberis amara, Menthae piperitae folium, Matricariae flos, Carvi fructus, Melissae folium, Angelicae radix, Liquiritiae radix, Cardui mariae fructus und Chelidonii herba in Form alkoholischer Extrakte enthält, gelöst, wobei in einem ersten Schritt a)Angelicae radix-Extrakt und Liquiritiae radix-Extrakt in einem Volumenverhältnis von 1:6 bis 3:1 miteinander vermischt werden und anschließend in mindestens einem weiteren Schritt die Mischung aus Schritt a) mit den Extrakten der weiteren Bestandteile oder sie enthaltenden Mischungen, welche separat erzeugt werden und gegebenenfalls nochmals Liquiritiae radix enthalten, zusammengegeben wird.

Vor dem Hintergrund der gestellten Aufgabe wurden eine Reihe von Prozessverfahren als auch analytischen Methoden untersucht oder speziell entwickelt. Insbesondere die flüchtigen Bestandteile der Drogen mit ätherischen Ölen und die Fällungen/Kristallbildung im Auszug aus Angelikawurzel führten zu nicht reproduzierbaren Qualitätsschwankungen. Es stellte sich überraschenderweise nur der Einsatz eines Herstellungsverfahrens gemäß Anspruch 1 als effektiv heraus. Entscheidend ist dabei die Zugabenfolge der einzelnen Drogenauszüge.

Eine bevorzugte Variante des erfindungsgemäßen Verfahrens zur Herstellung eines Arzneimittels auf pflanzlicher Basis ist **dadurch gekennzeichnet, dass** in einem Schritt b) eine Mischung enthaltend Extrakte von Matricariae flos, Iberis amara und Carvi fructus erzeugt wird, zu dieser die Mischung aus Schritt a) zugegeben wird und in einem Schritt c) Extrakte von Cardui mariae fructus, Menthae piperitae folium, Melissae folium und Chelidonii herba zugegeben werden. Der Schritt a) ist wie oben definiert, d.h. Angelicae radix-Extrakt und Liquiritiae radix-Extrakt werden in einem Volumenverhältnis von 1:6 bis 3:1 miteinander vermischt.

Es steht zu vermuten, dass die Saponine des Süßholzauszuges das Osthol im Angelica-Extrakt stabilisieren und dass sie im weiteren Verlauf die ätherischen Öle der überwiegend ölhaltigen Drogen emulgieren (Kamille, Iberis amara und Kümmel). Ferner wird angenommen, dass die schleimhaltigen Drogen wie Mariendistel die Sedimentation verhindern (Mariendistel, Minze, Melisse). Die Mischreihenfolge (Zugabenfolge) verhindert die Bildung von Kristallen der Inhaltsstoffe (sekundäre Pflanzenstoffe) der Kräuterauszüge. Die Kristallbildung beeinflusst die Chargenhomogenität. Daher sichert die erfindungsgemäße Zugabenfolge eine reproduzierbare Herstellung des Produktes.

Verwendet werden jeweils Einzelextrakte. Gegenüber Mehrtopfextrakten hat die Mischung von Einzelextrakten den Vorteil, dass für die jeweiligen Drogen und Frischpflanzen optimale Auszugsmittel und Extraktionsbedingungen ausgewählt werden können und so die wirksamkeitsbestimmenden bzw. pharmazeutisch relevanten Inhaltsstoffe in guter Ausbeute vorliegen. Die Übergangsraten der Inhaltsstoffe und die der Extraktivstoffausbeuten der eingesetzten Droge sind rückverfolgbar, was für die Analytik von großer Bedeutung ist.

Vorzugsweise werden in dem erfindungsgemäßen Verfahren zur Herstellung eines Arzneimittels auf pflanzlicher Basis Mischungen enthaltend Extrakte von Menthae piperitae folium, Carvi fructus und Melissae folium; Cardui mariae fructus und Matricariae flos; und Iberis amara und Chelidonii herba separat erzeugt.

Insbesondere ist es bevorzugt, dass Angelicae radix-Extrakt und Liquiritiae radix-Extrakt in Schritt a) in einem Volumenverhältnis von 1:2 bis 2:1 miteinander vermischt werden. In einer besonders bevorzugten Ausführungsform des erfindungsgemäßen Verfahrens werden Angelicae radix-Extrakt und Liquiritiae radix-Extrakt in Schritt a) in einem Volumenverhältnis von 1:1 miteinander vermischt.

Ausgehend von der initialen Vermischung von Angelicae radix-Extrakt und Liquiritiae radix-Extrakt in Schritt a) haben sich überraschenderweise verschiedene Varianten der weiteren Vermischung als besonders geeignet herausgestellt.

Eine bevorzugte Ausführungsform des erfindungsgemäßen Herstellungsverfahrens beruht darauf, dass in einem Schritt b) die Mischung aus Schritt a) mit einer Mischung enthaltend Extrakte von Menthae piperitae folium, Carvi fructus und Melissae folium zusammengegeben wird, in einem Schritt c) eine Mischung enthaltend Extrakte von Cardui mariae fructus und Matricariae flos zugegeben wird und in einem Schritt d) die Zugabe einer Mischung enthaltend Extrakte von Iberis amara und Chelidonii herba erfolgt.

Eine ebenfalls bevorzugte Ausführungsform ist **dadurch gekennzeichnet, dass** in einem Schritt b) die Mischung aus Schritt a) mit einer Mischung enthaltend Extrakte von Menthae piperitae folium, Carvi fructus und Melissae folium zusammengegeben wird, in einem Schritt e) eine Mischung enthaltend Extrakt von Cardui mariae fructus und Matricariae flos mit einer Mischung enthaltend Extrakte von Iberis amara und Chelidonii herba miteinander vermischt werden und in einem Schritt f) die Mischungen aus den Schritten b) und e) zusammengegeben werden.

Basierend auf einer initialen Vermischung von Angelicae radix-Extrakt und Liquiritiae radix-Extrakt in Schritt a) in einem Volumenverhältnis von 1:6 bis 3:1 hat es sich ebenfalls als bevorzugt erwiesen, einen bestimmten Anteil des Liquiritiae radix-Extrakts in einem späteren Schritt noch zusätzlich zuzugeben. Entsprechend ist eine bevorzugte Ausführungsform der vorliegenden Erfindung **dadurch gekennzeichnet, dass** die Menthae piperitae folium-Extrakt, Carvi fructus-Extrakt und Melissae folium-Extrakt enthaltende Mischung weiterhin Liquiritiae radix-Extrakt enthält. Dabei ist es besonders bevorzugt, wenn die Menthae piperitae folium-Extrakt, Carvi fructus-Extrakt und Melissae folium-Extrakt enthaltende Mischung weiterhin Liquiritiae radix-Extrakt enthält, wobei das Volumen des enthaltenden Liquiritiae radix-Extrakt im Verhältnis von 1:1 zu dem in der Mischung aus Angelicae radix-Extrakt und Liquiritiae radix-Extrakt aus Schritt a) enthaltenen Volumen an Liquiritiae radix-Extrakt steht.

Erfindungsgemäß ist das Herstellungsverfahren auf die Herstellung eines Arzneimittels auf pflanzlicher Basis gerichtet, welches
15 bis 40 Vol.-% Iberis amara,
5 bis 30 Vol.-% Menthae piperitae folium,
20 bis 40 Vol.-% Matricariae flos,
10 bis 30 Vol.-% Carvi fructus,
10 bis 30 Vol.-% Melissae folium,
5 bis 30 Vol.-% Angelicae radix,
10 bis 30 Vol.-% Liquiritiae radix,
5 bis 30 Vol.-% Cardui mariae fructus und
5 bis 30 Vol.-% Chelidonii herba in Form alkoholischer Extrakte umfasst. Bevorzugt ist das Verfahren auf die Herstellung eines Arzneimittels auf pflanzlicher Basis ausgerichtet, welches
15 Vol.-% Iberis amara,
5 Vol.-% Menthae piperitae folium,
20 Vol.-% Matricariae flos,
10 Vol.-% Carvi fructus,
10 Vol.-% Melissae folium,
10 Vol.-% Angelicae radix,
10 Vol.-% Liquiritiae radix,
10 Vol.-% Cardui mariae fructus und
10 Vol.-% Chelidonii herba in Form alkoholischer Extrakte umfasst.

Es ist bevorzugt, Extrakte aus Frischpflanzen oder Drogenextrakte zu verwenden. Insbesondere ist es vorzuziehen, wenn das Iberis amara-Extrakt ein Frischpflanzenextrakt aus Iberis amara totalis (Blüten, Blätter, Stengel und Wurzeln) ist. Die Ernte der Iberis amara Frischpflanze mit Blüten, Blätter, Stengel, Wurzeln erfolgt zu einem Zeitpunkt, an dem der Gehalt an Flavonoiden sein Optimum erreicht hat. Im Rahmen der Flavoniode sind die Glykoside des Kaempferols als Antiphlogistika besonders relevant. Der Auszug der Frischpflanze enthält als wichtigstes Flavonoid Kaempferol-3,4'-di-O-β-glucopyranosid-7-O-α-rhamnopyranosid. Die Frischpflanze enthält vorzugsweise mindestens 100 µg/g Flavonoide, insbesondere Kaempferol-3,4'-di-O-β-glucopyranosid-7-O-α-rhamnopyranosid. Hinsichtlich des Iberis amara-Frischpflanzenextraktes ist es bevorzugt, wenn dieser einen Flavonoid-Gehalt an Kaempferol-3,4'-di-O-β-glucopyranosid-7-O-α-rhamnopyranosid von 0,05 bis 0,2 mg/ml aufweist.

Das erfindungsgemäße Pflanzenmaterial enthält zudem einen begrenzten Gehalt an Cucurbitacinen, insbesondere an Cucurbitacin I und E. Die Frischpflanze enthält vorzugsweise einen Gehalt an Cucurbitacin I von maximal 500 µg/g und einen Gehalt an Cucurbitacin E von ebenfalls maximal 500 µg/g. Höhere Werte sind nicht erwünscht, da diese Stoffe ab einem gewissen Grenzwert Nebenwirkungspotential besitzen. Hinsichtlich des Extraktes ist es bevorzugt, dass das Iberis amara-Extrakt ein Frischpflanzenextrakt ist, welcher einen Gehalt an Cucurbitacinen von 0 bis 200 µg/ml aufweist. Dabei ist es besonders bevorzugt, wenn der Iberis amara-Extrakt ein Frischpflanzenextrakt ist, welcher einen Gehalt an Cucurbitacinen von 35 bis 185 µg/ml aufweist. Insbesondere ist es vorzuziehen, dass das Iberis amara-Extrakt ein Frischpflanzenextrakt ist, welcher einen Gehalt an Cucurbitacin I von 0 bis 100 µg/ml und einen Gehalt von Cucurbitacin E von 0 bis 100 µg/ml aufweist.

Wie bereits erwähnt, können Extrakte aus Frischpflanzen oder Drogenextrakte verwendet werden. Vorzugsweise ist das erfindungsgemäße Verfahren **dadurch gekennzeichnet, dass** die Extrakte von Menthae piperitae folium, Matricariae flos, Carvi fructus, Melissae folium, Angelicae radix, Liquiritiae radix, Cardui mariae fructus und Chelidonii herba Drogenextrakte sind.

Hinsichtlich des Frischpflanzenauszuges aus Iberis amara totalis ist es vorzuziehen, dass das Verhältnis von mazerierter/perkolierter Pflanze zum Auszug zwischen 1 Gramm: 10 ml (10 Gramm) und 1 Gramm : 1 ml (1 Gramm) beträgt. Insbesondere ist es bevorzugt, dass bei dem Frischpflanzenextrakt das Verhältnis von mazerierter/perkolierter Pflanze zum Extrakt von 1 Gramm : 1,5 ml (1,5 Gramm) bis 1 Gramm : 2,5 ml (2,5 Gramm) beträgt.

Vorzugsweise beträgt bei den Drogenextrakten das Verhältnis von Drogen zum Extrakt von 1 Gramm : 1 ml (1 Gramm) bis 1 Gramm : 10 ml (10 Gramm). Dabei ist es besonders bevorzugt, wenn bei den Drogenextrakten das Verhältnis von Drogen zum Auszug von 1 Gramm : 2 ml (2 Gramm) bis 1 Gramm : 4 ml (4 Gramm) beträgt. In einer bevorzugten Ausführungsform beträgt das Verhältnis bei den Drogenextrakten von Drogen zum Extrakt von 1 Gramm : 2,5 ml (2,5 Gramm) bis 1 Gramm : 3,5 ml (3,5 Gramm).

Das bevorzugte Extraktionsmittel umfasst Wasser und wässriges Ethanol mit einer Konzentration von 0.1 bis 60 Vol.-% Ethanol. Besonders bevorzugt umfasst das Extraktionsmittel wässriges Ethanol mit einer Konzentration von 30 bis 50 Vol.-% Ethanol. In einer bevorzugten Ausführungsform ist das Extraktionsmittel für den Frischpflanzenextrakt wässriges Ethanol mit einer Konzentration von 50 Vol.-% Ethanol und das Extraktionsmittel für die Drogenextrakte wässriges Ethanol mit einer Konzentration von 30 Vol.-% Ethanol.

Die vorliegende Erfindung umfasst auch ein Arzneimittel auf pflanzlicher Basis, welches nach dem erfindungsgemäßen Verfahren hergestellt wurde.

Ebenso umfasst die vorliegende Erfindung die Verwendung dieses Arzneimittels auf pflanzlicher Basis zur Behandlung von Beschwerden des Magen-Darm-Traktes, insbesondere zur Behandlung der funktionellen Dyspepsie.

Weiterhin ist die Verwendung des Arzneimittels auf pflanzlicher Basis zur Hemmung der Magensaftproduktion bevorzugt, sowie die Verwendung zur Behandlung von Geschwüren.

Bevorzugt wird das Arzneimittel als Anti-Inflammatikum und/oder Anti-Phlogistikum verwendet. Ebenso ist die Verwendung als Antioxidans und/oder Radikalfänger bevorzugt.

Eine weitere bevorzugte Verwendung stellt der Einsatz des erfindungsgemäßen Arzneimittels als Modulator der gastrointestinalen Mobilität dar.

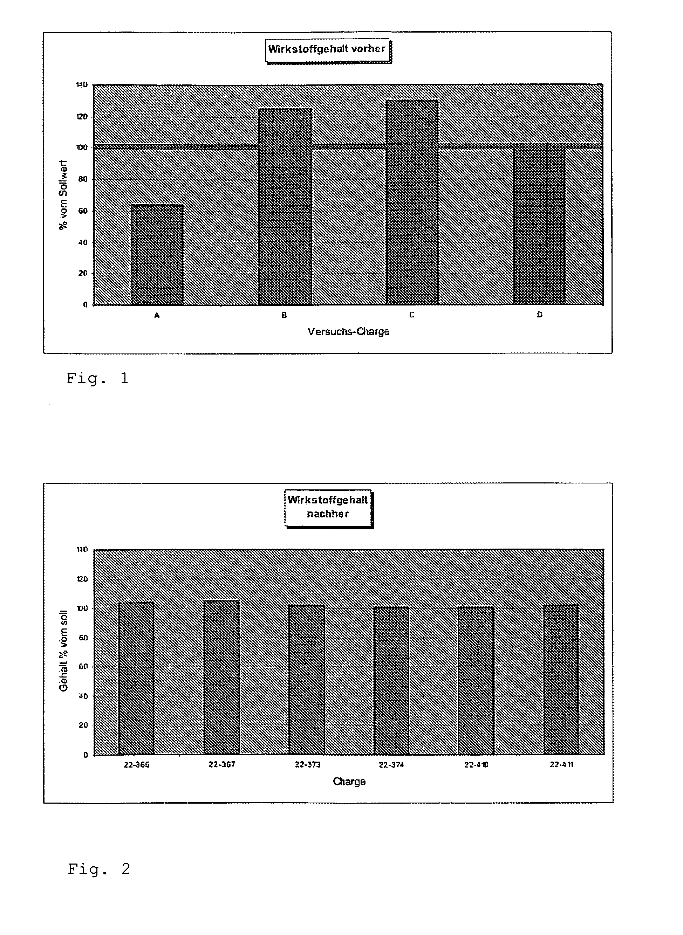

Bei Einhaltung der erfindungsgemäßen Mischungsreihenfolge wird die Kristallbildung signifikant unterdrückt. Ohne die Einhaltung dieser Reihenfolge liegen die Gehaltswerte für Osthol sowohl im Angelika-Drogenextrakt als auch im Fertigprodukt weit außerhalb der zulässigen Toleranz, d.h. die Wiederfindung der Leitsubstanz Osthol lag nicht im Bereich 95 bis 105% des Sollwertes. Die unter Einhaltung der erfindungsgemäßen Mischungsreihenfolge produzierten Chargen wiesen keine Abweichungen mehr auf. Ebenso ist ersichtlich, dass bei Einhaltung der Reihenfolge kaum noch Kristallbildung zu verzeichnen ist. Beispielhaft zeigen die Fig. 1 und 2 die Chargenhomogenität hinsichtlich des untersuchten Pflanzeninhaltstoffes (Osthol) bei beliebiger Reihenfolge (Fig. 1) und mit erfindungsgemäßer Mischreihenfolge der einzelnen Kräuterauszüge gemäß den Beispielen 1, 2 und 3 (Fig. 2). Die erfindungsgemäßen Beispiele 1, 2 und 3 zeigten keine nennenswerten Unterschiede, so dass auf eine separate Darstellung verzichtet wurde.

Nachfolgend wird die Erfindung anhand von Beispielen weiter erläutert.

### BEISPIELE

### Herstellungsformel

| **Bestandteile** | **Menge in g** | **Menge in ml** |
|---|---|---|
| Drogenextrakt aus Kamillenblüten (1 : 2 - 4) | 1980 | 2000 |
| Frischpflanzenextrakt aus Schleifenblume, bittere (1 : 1,5 - 2,5) | 1455 | 1500 |
| Drogenextrakt aus Kümmel (1 : 2,5 - 3,5) | 975 | 1000 |
| Mischung aus Drogenextrakt aus Süßholzwurzeln (1 : 2, | 990 | 1000 |
| 5 - 3,5), Drogenextrakt aus Angelikawurzeln (1 : 2,5 - 3,5) | 990 | 1000 |
| Drogenextrakt aus Mariendistelfrüchten (1 : 2,5- 3,5) | 970 | 1000 |
| Drogenextrakt aus Pfefferminzblättern (1 : 2,5 - 3, 5) | 495 | 500 |
| Drogenextrakt aus Melissenblättern (1 : 2,5 - 3, 5) | 990 | 1000 |
| Drogenextrakt aus Schöllkraut (1 : 2,5 - 3,5) | 990 | 1000 |

Auszugsmittel für Iberis amara: Ethanol 50% (V/V)
Auszugsmittel für alle anderen Drogenextrakte: Ethanol 30% (V/V)

Die einzelnen Bestandteile müssen vor der Verwiegung homogen gemischt werden. Die Bestandteile werden in Edelstahl- oder Kunststoffbehälter nacheinander gemäß Herstellungsformel eingewogen und gemischt.

### Beispiel 1 - Reihenfolge A

Vormischung: Es wird eine Vormischung aus Süßholzwurzel-Extrakt und Angelikawurzel-Extrakt im Verhältnis 1:1 hergestellt. Die Bestandteile werden gemäß Herstellungsformel nacheinander eingewogen und gemischt.

Endmischung: Die Bestandteile Pfefferminzblätter-Drogenextrakt, Kümmel-Drogenextrakt und Melissen-Drogenextrakt werden vermischt. Anschließend wird die Vormischung dazugegeben. Die Bestandteile Kamillen-Drogenextrakt und Mariendistel-Drogenextrakt, sowie Schöllkraut-Drogenextrakt und Frischpflanzenextrakt Schleifenblume werden dazu eingewogen. Die Extrakte werden gemischt.

### Beispiel 2 - Reihenfolge B

Vormischung: Angelicawurzel-Extrakt und Süßholzwurzel-Extrakt werden 1:1 gemischt.

Endmischung: Die Bestandteile Pfefferminzblätter-Drogenextrakt, Kümmel-Drogenextrakt und Melissen-Drogenextrakt werden vermischt. Zu dieser Mischung wird die Mischung aus Angelicawurzel-Extrakt und Süßholzwurzel-Extrakt zugegeben. Separat wird eine Mischung aus Mariendistelfrüchte-Drogenextrakt und Kamillen-Drogenextrakt mit einer Mischung aus Schöllkraut-Drogenextrakt und Frischpflanzenextrakt Schleifenblume erzeugt. Die Mischungen werden abschließend miteinander vermischt.

### Beispiel 3 - Reihenfolge C

Vormischung: Es wird eine Vormischung aus Süßholzwurzel-Extrakt und Angelikawurzel-Extrakt im Verhältnis 1:2 (1 Teil : 2 Teile) hergestellt. Die Bestandteile werden gemäß Herstellungsformel nacheinander eingewogen und gemischt.

Endmischung: Ein weiterer Teil Süßholzwurzel-Extrakt wird mit Pfefferminzblätter-Drogenextrakt, Melissenblätter-Drogenextrakt und Kümmel-Drogenextrakt zusammengegeben. Die erhaltene Mischung wird mit der Vormischung versetzt. Anschließend werden Kamillen-Drogenextrakt und Mariendistel-Drogenextrakt sowie Frischpflanzenextrakt Schleifenblume und Schöllkraut-Drogenextrakt zugegeben.

Die erhaltenen Lösungen aus den Beispielen 1, 2 und 3 werden in Edelstahl- oder Kunststofftanks bei Raumtemperatur (15 - 25°C) mindestens 14 Tage ( einschließlich Einlagerungs- und Auslagerungstag) gelagert.

Der Ostholgehalt wurde bestimmt hinsichtlich Proben, welche nach den Mischungsreihenfolgen aus den Beispielen 1, 2 und 3 hergestellt wurden. Es wurde mit Proben, welche auf einer beliebigen Mischreihenfolge der einzelnen Kräuterauszüge basierten, verglichen. Die Ergebnisse der Messungen sind in den Figuren 1 (beliebige Mischreihenfolge, ohne Vormischung) und 2 (erfindungsgemäße Mischreihenfolgen) dargestellt. Wie aus der Fig. 2 ersichtlich ist, verhindert die Mischreihenfolge die Bildung von Kristallen der Inhaltsstoffe (sekundäre Pflanzenstoffe) der Kräuterauszüge.

Mikroskopisch wurde die Kristallbildung untersucht, wobei die Messungen nach 2 beziehungsweise 6 Wochen Lagerung erfolgten. Die Ergebnisse sind in den Tabellen 1 und 2 dargestellt.

**Tabelle 1: Lagerung 2 Wochen bei Raumtemperatur**

| Beispiel 1 | Beispiel 2 | Beispiel 3 |
|---|---|---|
| Bodensatz leicht, dunkel, pulvrig; Überstand kolloidal, sehr trüb; | Bodensatz dünn dunkel, pulvrig; Überstand kolloidal, sehr trüb; | Bodensatz dünn dunkel, pulvrig; Überstand kolloidal, sehr trüb; |
| gleichmäßige Partikel von ca. 2 µm, vereinzelte kleine Kristalle bis 2µm | gleichmäßige Partikel von ca. 2 µm, zum Teil zusammengeballt; vereinzelte kleine Kristalle ca. 1 µm | gleichmäßige Partikel von ca. 2 µm, einige wenige zusammengeballt; keine Kristalle |

**Tabelle 2: Lagerung 6 Wochen bei Raumtemperatur**

| Beispiel 1 | Beispiel 2 | Beispiel 3 |
|---|---|---|
| Bodensatz leicht dunkel, pulvrig; Überstand kolloidal leicht trüb; Bodensatz aus gelartig verbundenen, gleichmäßigen Partikeln von ca. 2 µm, viele kleine Kristalle bis 4 µm | Bodensatz leicht dunkel, pulvrig; Überstand kolloidal, leicht trüb; gleichmäßige Partikel von ca. 2 µm, z.T. schleimartig zusammengeballt, vereinzelte kleine Kristalle ca. 1 µm, wenige bis 2 µm | Bodensatz leicht dunkel, pulvrig; Überstand schwach kolloidal, kaum trüb; gleichmäßige Partikel von ca. 2 µm, einige wenige zusammengeballt, viele ganz kleine Kristalle 1 µm, wenige Nadeln bis 2 µm |

## Patentansprüche

1. Verfahren zur Herstellung eines Arzneimittels auf pflanzlicher Basis, welches Iberis amara, Menthae piperitae folium, Matricariae flos, Carvi fructus, Melissae folium, Angelicae radix, Liquiritiae radix, Cardui mariae fructus und Chelidonii herba in Form alkoholischer Extrakte enthält, **dadurch gekennzeichnet, dass** in einem ersten Schritt
a)Angelicae radix-Extrakt und Liquiritiae radix-Extrakt in einem Volumenverhältnis von 1:6 bis 3:1 miteinander vermischt werden und
anschließend in mindestens einem weiteren Schritt die Mischung aus Schritt a) mit den Extrakten der weiteren Bestandteile oder sie enthaltenden Mischungen, welche separat erzeugt werden und gegebenenfalls nochmals Liquiritiae radix enthalten, zusammengegeben wird.

2. Verfahren zur Herstellung eines Arzneimittels auf pflanzlicher Basis nach Anspruch 1, **dadurch gekennzeichnet, dass** in einem Schritt b) eine Mischung enthaltend Extrakte von Matricariae flos, Iberis amara und Carvi fructus erzeugt wird, zu dieser die Mischung aus Schritt a) zugegeben wird und in einem Schritt c) Extrakte von Cardui mariae fructus, Menthae piperitae folium, Melissae folium und Chelidonii herba zugegeben werden.

3. Verfahren zur Herstellung eines Arzneimittels auf pflanzlicher Basis nach.Anspruch 1 oder 2, **dadurch gekennzeichnet, dass** Mischungen enthaltend Extrakte von Menthae piperitae folium, Carvi fructus und Melissae folium; Cardui mariae fructus und Matricariae flos; und Iberis amara und Chelidonii herba separat erzeugt werden.

4. Verfahren zur Herstellung eines Arzneimittels auf pflanzlicher Basis nach einem der Ansprüche 1 bis 3, **dadurch gekennzeichnet, dass** Angelicae radix-Extrakt und Liquiritiae radix-Extrakt in Schritt a) in einem Volumenverhältnis von 1:2 bis 2:1 miteinander vermischt werden.

5. Verfahren zur Herstellung eines Arzneimittels auf pflanzlicher Basis nach einem der Ansprüche 1 bis 4, **dadurch gekennzeichnet, dass** Angelicae radix-Extrakt und Liquiritiae radix-Extrakt in Schritt a) in einem Volumenverhältnis von 1:1 miteinander vermischt werden.

6. Verfahren zur Herstellung eines Arzneimittels auf pflanzlicher Basis nach einem der Ansprüche 1 bis 5, **dadurch gekennzeichnet, dass** in einem Schritt b) die Mischung aus Schritt a) mit einer Mischung enthaltend Extrakte von Menthae piperitae folium, Carvi fructus und Melissae folium zusammengegeben wird, in einem Schritt c) eine Mischung enthaltend Extrakte von Cardui mariae fructus und Matricariae flos zugegeben wird und in einem Schritt d) die Zugabe einer Mischung enthaltend Extrakte von Iberis amara und Chelidonii herba erfolgt.

7. Verfahren zur Herstellung eines Arzneimittels auf pflanzlicher Basis nach einem der Ansprüche 1 bis 6, **dadurch gekennzeichnet, dass** in einem Schritt b) die Mischung aus Schritt a) mit einer Mischung enthaltend Extrakte von Menthae piperitae folium, Carvi fructus und Melissae folium zusammengegeben wird, in einem Schritt e) eine Mischung enthaltend Extrakt von Cardui mariae fructus und Matricariae flos mit einer Mischung enthaltend Extrakte von Iberis amara und Chelidonii herba miteinander vermischt werden und in einem Schritt f) die Mischungen aus den Schritten b) und e) zusammengegeben werden.

8. Verfahren zur Herstellung eines Arzneimittels auf pflanzlicher Basis nach einem der Ansprüche 1 bis 7, **dadurch gekennzeichnet, dass** die Menthae piperitae folium-Extrakt, Carvi fructus-Extrakt und Melissae folium-Extrakt enthaltende Mischung weiterhin Liquiritiae radix-Extrakt enthält.

9. Verfahren zur Herstellung eines Arzneimittels auf pflanzlicher Basis nach einem der Ansprüche 1 bis 8, **dadurch gekennzeichnet, dass** die Menthae piperitae folium-Extrakt, Carvi fructus-Extrakt und Melissae folium-Extrakt enthaltende Mischung weiterhin Liquiritiae radix-Extrakt enthält, wobei das Volumen des enthaltenden Liquiritiae radix-Extrakt im Verhältnis von 1:1 zu dem in der Mischung aus Angelicae radix-Extrakt und Liquiritiae radix-Extrakt aus Schritt a) enthaltenen Volumen an Liquiritiae radix-Extrakt steht.

10. Verfahren zur Herstellung eines Arzneimittels auf pflanzlicher Basis nach einem der Ansprüche 1 bis 9, welches
15 bis 40 Vol.-% Iberis amara,
5 bis 30 Vol.-% Menthae piperitae folium,
20 bis 40 Vol.-% Matricariae flos,
10 bis 30 Vol.-% Carvi fructus,
10 bis 30 Vol.-% Melissae folium,
5 bis 30 Vol.-% Angelicae radix,
10 bis 30 Vol.-% Liquiritiae radix,
5 bis 30 Vol.-% Cardui mariae fructus und
5 bis 30 Vol.-% Chelidonii herba in Form alkoholischer Extrakte umfasst.

11. Verfahren zur Herstellung eines Arzneimittels auf pflanzlicher Basis nach einem der Ansprüche 1 bis 10, welches
15 Vol.-% Iberis amara,
5 Vol.-% Menthae piperitae folium,
20 Vol.-% Matricariae flos,
10 Vol.-% Carvi fructus,
10 Vol.-% Melissae folium,
10 Vol.-% Angelicae radix,
10 Vol.-% Liquiritiae radix,
10 Vol.-% Cardui mariae fructus und
10 Vol.-% Chelidonii herba in Form alkoholischer Extrakte umfasst.

12. Verfahren zur Herstellung eines Arzneimittels auf pflanzlicher Basis nach einem der Ansprüche 1 bis 11, **dadurch gekennzeichnet, dass** Extrakte aus Frischpflanzen oder Drogenextrakte verwendet werden.

13. Verfahren zur Herstellung eines Arzneimittels auf pflanzlicher Basis nach einem der Ansprüche 1 bis 12, **dadurch gekennzeichnet, dass** das Iberis amara-Extrakt ein Frischpflanzenextrakt aus Iberis amara totalis (Blüten, Blätter, Stengel und Wurzeln) ist.

14. Verfahren zur Herstellung eines Arzneimittels auf pflanzlicher Basis nach einem der Ansprüche 1 bis 13, **dadurch gekennzeichnet, dass** der Iberis amara-Extrakt ein Frischpflanzenextrakt ist, welcher einen Flavonoid-Gehalt von 0,05 bis 0,2 mg/ml aufweist.

15. Verfahren zur Herstellung eines Arzneimittels auf pflanzlicher Basis nach einem der Ansprüche 1 bis 14, **dadurch gekennzeichnet, dass** der Iberis amara-Extrakt ein Frischpflanzenextrakt ist, welcher einen Gehalt an Cucurbitacinen von 0 bis 200 µg/ml aufweist.

16. Verfahren zur Herstellung eines Arzneimittels auf pflanzlicher Basis nach einem der Ansprüche 1 bis 15, **dadurch gekennzeichnet, dass** das Iberis amara-Extrakt ein Frischpflanzenextrakt ist, welcher einen Gehalt an Cucurbitacinen von 35 bis 185 µg/ml aufweist.

17. Verfahren zur Herstellung eines Arzneimittels auf pflanzlicher Basis nach einem der Ansprüche 1 bis 16, **dadurch gekennzeichnet, dass** der Iberis amara-Extrakt ein Frischpflanzenextrakt ist, welcher einen Gehalt an Cucurbitacin I von 0 bis 100 µg/ml und einen Gehalt von Cucurbitacin E von 0 bis 100 µg/ml aufweist.

18. Verfahren zur Herstellung eines Arzneimittels auf pflanzlicher Basis nach einem der Ansprüche 1 bis 17, **dadurch gekennzeichnet, dass** die Extrakte von Menthae piperitae folium, Matricariae flos, Carvi fructus, Melissae folium, Angelicae radix, Liquiritiae radix, Cardui mariae fructus und Chelidonii herba Drogenextrakte sind.

19. Verfahren zur Herstellung eines Arzneimittels auf pflanzlicher Basis nach einem der Ansprüche 1 bis 18, **dadurch gekennzeichnet, dass** bei dem Frischpflanzenextrakt das Verhältnis von mazerierter/perkolierter Pflanze zum Auszug zwischen 1 Gramm: 10 ml (10 Gramm) und 1 Gramm : 1 ml (1 Gramm) beträgt.

20. Verfahren zur Herstellung eines Arzneimittels auf pflanzlicher Basis nach einem der Ansprüche 1 bis 19, **dadurch gekennzeichnet, dass** bei dem Frischpflanzenextrakt das Verhältnis von mazerierter/perkolierter Pflanze zum Extrakt von 1 Gramm **:** 1,5 ml (1,5 Gramm) bis 1 Gramm : 2,5 ml (2,5 Gramm) beträgt.

21. Verfahren zur Herstellung eines Arzneimittels auf pflanzlicher Basis nach einem der Ansprüche 1 bis 20, **dadurch gekennzeichnet, dass** bei dem Drogenextrakt das Verhältnis von Drogen zum Extrakt von 1 Gramm : 1 ml (1 Gramm) bis 1 Gramm : 10 ml (10 Gramm) beträgt.

22. Verfahren zur Herstellung eines Arzneimittels auf pflanzlicher Basis nach einem der Ansprüche 1 bis 21, **dadurch gekennzeichnet, dass** bei dem Drogenextrakt das Verhältnis von Drogen zum Auszug von 1 Gramm : 2 ml (2 Gramm) bis 1 Gramm : 4 ml (4 Gramm) beträgt.

23. Verfahren zur Herstellung eines Arzneimittels auf pflanzlicher Basis nach einem der Ansprüche 1 bis 22, **dadurch gekennzeichnet, dass** bei dem Drogenextrakt das Verhältnis von Drogen zum Extrakt von 1 Gramm : 2,5 ml (2,5 Gramm) bis 1 Gramm : 3,5 ml (3,5 Gramm) beträgt.

24. Verfahren zur Herstellung eines Arzneimittels auf pflanzlicher Basis nach einem der Ansprüche 1 bis 23, **dadurch gekennzeichnet, dass** das Extraktionsmittel Wasser und wässriges Ethanol mit einer Konzentration von 0.1 bis 60 Vol.-% Ethanol umfasst.

25. Verfahren zur Herstellung eines Arzneimittels auf pflanzlicher Basis nach einem der Ansprüche 1 bis 24, **dadurch gekennzeichnet, dass** das Extraktionsmittel wässriges Ethanol mit einer Konzentration von 30 bis 50 Vol.-% Ethanol umfasst.

26. Verfahren zur Herstellung eines Arzneimittels auf pflanzlicher Basis nach einem der Ansprüche 1 bis 25, **dadurch gekennzeichnet, dass** das Extraktionsmittel für den Frischpflanzenextrakt wässriges Ethanol mit einer Konzentration von 50 Vol.-% Ethanol und das Extraktionsmittel für die Drogenextrakte wässriges Ethanol mit einer Konzentration von 30 Vol.-% Ethanol ist.

## Claims

1. A method for producing a plant-based medicine which contains Iberis amara, Menthae piperitae folium, Matricariae flos, Carvi fructus, Melissae folium, Angelicae radix, Liquiritiae radix, Cardui mariae fructus and Chelidonii herba in the form of alcoholic extracts, **characterised in that** in a first step
a) Angelicae radix extract and Liquiritiae radix extract are mixed together in a volume ratio of 1:6 to 3:1 and
subsequently in at least one further step the mixture from step a) is mixed together with the extracts of the further constituents or mixtures containing same, which are produced separately and where applicable additionally contain Liquiritiae radix.

2. The method for producing a plant-based medicine according to Claim 1, **characterised in that** in a step b) a mixture containing extracts of Matricariae flos, Iberis amara and Carvi fructus is produced, the mixture from step a) is added thereto, and in a step c) extracts of Cardui mariae fructus, Menthae piperitae folium, Melissae folium and Chelidonii herba are added.

3. The method for producing a plant-based medicine according to Claim 1 or 2, **characterised in that** mixtures containing extracts of Menthae piperitae folium, Carvi fructus and Melissae folium; Cardui mariae fructus and Matricariae flos; and Iberis amara and Chelidonii herba are produced separately.

4. The method for producing a plant-based medicine according to one of Claims 1 to 3, **characterised in that** Angelicae radix extract and Liquiritiae radix extract are mixed together in step a) in a volume ratio from 1:2 to 2:1.

5. The method for producing a plant-based medicine according to one of Claims 1 to 4, **characterised in that** Angelicae radix extract and Liquiritiae radix extract are mixed together in step a) in a volume ratio of 1:1.

6. The method for producing a plant-based medicine according to one of Claims 1 to 5, **characterised in that** in a step b) the mixture of step a) is mixed together with a mixture containing extracts of Menthae piperitae folium, Carvi fructus and Melissae folium, in a step c) a mixture containing extracts of Cardui mariae fructus and Matricariae flos is added, and in a step d) a mixture containing extracts of Iberis amara and Chelidonii herba is added.

7. The method for producing a plant-based medicine according to one of Claims 1 to 6, **characterised in that** in a step b) the mixture of step a) is mixed together with a mixture containing extracts of Menthae piperitae folium, Carvi fructus and Melissae folium, in a step e) a mixture containing extract of Cardui mariae fructus and Matricariae flos is mixed together with a mixture containing extracts of Iberis amara and Chelidonii herba, and in a step f) the mixtures from the steps b) and e) are mixed together.

8. Method for producing a plant-based medicine according to one of Claims 1 to 7, **characterised in that** the mixture containing Menthae piperitae folium extract, Carvi fructus extract and Melissae folium extract additionally contains Liquiritiae radix extract.

9. The method for producing a plant-based medicine according to one of Claims 1 to 8, **characterised in that** the mixture containing Menthae piperitae folium extract, Carvi fructus extract and Melissae folium extract additionally contains Liquiritiae radix extract, wherein the volume of the contained Liquiritiae radix extract is in the ratio of 1:1 to the volume of Liquiritiae radix extract contained in the mixture of Angelicae radix extract and Liquiritiae radix extract from step a).

10. The method for producing a plant-based medicine according to one of Claims 1 to 9, which comprises
15 to 40 vol. % Iberis amara,
5 to 30 vol. % Menthae piperitae folium,
20 to 40 vol. % Matricariae flos,
10 to 30 vol. % Carvi fructus,
10 to 30 vol. % Melissae folium,
5 to 30 vol. % Angelicae radix,
10 to 30 vol. % Liquiritiae radix,
5 to 30 vol. % Cardui mariae fructus and
5 to 30 vol. % Chelidonii herba in the form of alcoholic extracts.

11. The method for producing a plant-based medicine according to one of Claims 1 to 10, which comprises
15 vol. % Iberis amara,
5 vol. % Menthae piperitae folium,
20 vol. % Matricariae flos,
10 vol. % Carvi fructus,
10 vol. % Melissae folium,
10 vol. % Angelicae radix,
10 vol. % Liquiritiae radix,
10 vol. % Cardui mariae fructus and
10 vol. % Chelidonii herba in the form of alcoholic extracts.

12. The method for producing a plant-based medicine according to one of Claims 1 to 11, **characterised in that** extracts of fresh plants or drug extracts are used.

13. The method for producing a plant-based medicine according to one of Claims 1 to 12, **characterised in that** the Iberis amara extract is a fresh plant extract of Iberis amara totalis (flowers, leaves, stem and roots).

14. The method for producing a plant-based medicine according to one of Claims 1 to 13, **characterised in that** the Iberis amara extract is a fresh plant extract which has a flavonoid content from 0.05 to 0.2 mg/ml.

15. The method for producing a plant-based medicine according to one of Claims 1 to 14, **characterised in that** the Iberis amara extract is a fresh plant extract which has a content of cucurbitacins from 0 to 200 µg/ml.

16. The method for producing a plant-based medicine according to one of Claims 1 to 15, **characterised in that** the Iberis amara extract is a fresh plant extract which has a content of cucurbitacins from 35 to 185 µg/ml.

17. The method for producing a plant-based medicine according to one of Claims 1 to 16, **characterised in that** the Iberis amara extract is a fresh plant extract which has a content of cucurbitacin I from 0 to 100 µg/ml and a content of cucurbitacin E from 0 to 100 µg/ml.

18. The method for producing a plant-based medicine according to one of Claims 1 to 17, **characterised in that** the extracts of Menthae piperitae folium, Matricariae flos, Carvi fructus, Melissae folium, Angelicae radix, Liquiritiae radix, Cardui mariae fructus and Chelidonii herba are drug extracts.

19. The method for producing a plant-based medicine according to one of Claims 1 to 18, **characterised in that** in the fresh plant extract, the ratio of macerated/percolated plant to the extract is between 1 gram:10 ml (10 gram) and 1 gram:1 ml (1 gram).

20. The method for producing a plant-based medicine according to one of Claims 1 to 19, **characterised in that** in the fresh plant extract, the ratio of macerated/percolated plant to the extract is from 1 gram:1.5 ml (1.5 gram) to 1 gram:2.5 ml (2.5 gram).

21. The method for producing a plant-based medicine according to one of Claims 1 to 20, **characterised in that** in the drug extract, the ratio of drugs to the extract is from 1 gram:1 ml (1 gram) to 1 gram: 10 ml (10 gram).

22. The method for producing a plant-based medicine according to one of Claims 1 to 21, **characterised in that** in the drug extract, the ratio of drugs to the extract is 1 gram:2 ml (2 gram) to 1 gram: 4 ml (4 gram).

23. The method for producing a plant-based medicine according to one of Claims 1 to 22, **characterised in that** in the drug extract, the ratio of drugs to the extract is from 1 gram:2.5 ml (2.5 gram) to 1 gram:3.5 ml (3.5 gram).

24. The method for producing a plant-based medicine according to one of Claims 1 to 23, **characterised in that** the extractant comprises water and aqueous ethanol with a concentration from 0.1 to 60 vol. % ethanol.

25. The method for producing a plant-based medicine according to one of Claims 1 to 24, **characterised in that** the extractant comprises aqueous ethanol with a concentration from 30 to 50 vol. %.

26. The method for producing a plant-based medicine according to one of Claims 1 to 25, **characterised in that** the extractant for the fresh plant extract is aqueous ethanol with a concentration of 50 vol. % ethanol, and the extractant for the drug extracts is aqueous ethanol with a concentration of 30 vol. % ethanol.

## Revendications

1. Procédé de fabrication d'un médicament à base de plantes contenant des extraits alcooliques d'Iberis amara, Menthae piperitae folium, Matricariae flos, Carvi fructus, Melissae folium, Angelicae radix, Liquiritiae radix, Cardui mariae fructus et de Chelidonii herba, **caractérisé en ce que**, dans une première étape, a) on mélange l'extrait d'Angelicae radix et l'extrait de Liquiritiae radix dans un rapport volumique compris entre 1 : 6 et 3 : 1, pour ensuite, dans au moins une étape ultérieure, réunir le mélange obtenu à l'étape a) avec les extraits des autres ingrédients ou avec des mélanges les contenant, ces derniers ayant été produits séparément et lesquels pouvant éventuellement contenir des quantités supplémentaires de Liquiritiae radix.

2. Procédé de fabrication d'un médicament à base de plantes selon la revendication 1, **caractérisé en ce que**, dans une étape b), on produit un mélange contenant des extraits de Matricariae flos, Iberis amara et de Carvi fructus, pour y ajouter le mélange obtenu à l'étape a) puis, dans une étape c), ajouter des extraits de Cardui mariae fructus, Menthae piperitae folium, Melissae folium et de Chelidonii herba.

3. Procédé de fabrication d'un médicament à base de plantes selon les revendications 1 ou 2, **caractérisé en ce que** l'on produit séparément des mélanges contenant des extraits de Menthae piperitae folium, Carvi fructus et de Melissae folium ; de Cardui mariae fructus et de Matricariae flos ; et d'Iberis amara et de Chelidonii herba.

4. Procédé de fabrication d'un médicament à base de plantes, selon l'une des revendications 1 à 3, **caractérisé en ce que**, dans l'étape a), l'extrait d'Angelicae radix et l'extrait de Liquiritiae radix sont mélangés dans un rapport volumique compris entre 1 : 2 et 2 : 1.

5. Procédé de fabrication d'un médicament à base de plantes, selon l'une des revendications 1 à 4, **caractérisé en ce que**, dans l'étape a), l'extrait d'Angelicae radix et l'extrait de Liquiritiae radix sont mélangés dans un rapport volumique égal à 1 : 1.

6. Procédé de fabrication d'un médicament à base de plantes selon l'une des revendications 1 à 5, **caractérisé en ce que**, dans une étape b), on réunit le mélange obtenu à l'étape a) avec un mélange contenant des extraits de Menthae piperitae folium, de Carvi fructus et de Melissae folium, pour y ajouter, dans une étape c), un mélange contenant des extraits de Cardui mariae fructus et de Matricariae flos puis y ajouter, dans une étape d), un mélange contenant des extraits d'Iberis amara et de Chelidonii herba.

7. Procédé de fabrication d'un médicament à base de plantes selon l'une des revendications 1 à 6, **caractérisé en ce que** l'on réunit, dans une étape b), le mélange obtenu à l'étape a) avec un mélange contenant les extraits de Menthae piperitae folium, de Carvi fructus et de Melissae folium, que d'on mélange, dans une étape e), un mélange contenant les extraits de Cardui mariae fructus et de Matricariae flos avec un mélange contenant des extraits d'Iberis amara et de Chelidonii herba pour réunir, dans une étape f), les mélanges obtenus aux étapes b) et e).

8. Procédé de fabrication d'un médicament à base de plantes selon l'une des revendications 1 à 7, **caractérisé en ce que** le mélange contenant de l'extrait de Menthae piperitae folium, de l'extrait de Carvi fructus et de l'extrait de Melissae folium, contient en outre de l'extrait de Liquiritiae radix.

9. Procédé de fabrication d'un médicament à base de plantes selon l'une des revendications 1 à 8, **caractérisé en ce que** le mélange contenant de l'extrait de Menthae piperitae folium, de l'extrait de Carvi fructus et de l'extrait de Melissae folium, contient en outre de l'extrait de Liquiritiae radix, le rapport entre le volume de l'extrait de Liquiritiae radix contenu dans ce dernier et le volume de l'extrait de Liquiritiae radix contenu dans le mélange d'extrait d'Angelicae radix et d'extrait de Liquiritiae radix étant égal 1 : 1.

10. Procédé de fabrication d'un médicament à base de plantes selon l'une des revendications 1 à 9, lequel comprend
15 à 40 % en volume d'Iberis amara,
5 à 30 % en volume de Menthae piperitae folium,
20 à 40 % en volume de Matricariae flos,
10 à 30 % en volume de Carvi fructus,
10 à 30 % en volume de Melissae folium,
5 à 30 % en volume d'Angelicae radix,
10 à 30 % en volume de Liquiritiae radix,
5 à 30 % en volume de Cardui mariae fructus et
5 à 30 % en volume de Chelidonii herba sous forme d'extraits alcooliques.

11. Procédé de fabrication d'un médicament à base de plantes selon l'une des revendications 1 à 10, lequel comprend
15 % en volume d'Iberis amara,
5 % en volume de Menthae piperitae folium,
20 % en volume de Matricariae flos,
10 % en volume de Carvi fructus,
10 % en volume de Melissae folium,
10 % en volume d'Angelicae radix,
10 % en volume de Liquiritiae radix,
10 % en volume de Cardui mariae fructus et
10 % en volume de Chelidonii herba sous forme d'extraits alcooliques.

12. Procédé de fabrication d'un médicament à base de plantes selon l'une des revendications 1 à 11, **caractérisé en ce que** l'on utilise des extraits de plantes fraîches ou des extraits de plantes séchées.

13. Procédé de fabrication d'un médicament à base de plantes selon l'une des revendications 1 à 12, **caractérisé en ce que** l'extrait d'Iberis amara est un extrait de plantes fraîches obtenu à partir d'Iberis amara totalis (fleurs, feuilles, tiges et racines).

14. Procédé de fabrication d'un médicament à base de plantes selon l'une des revendications 1 à 13, **caractérisé en ce que** l'extrait d'Iberis amara est un extrait de plantes fraîches comportant une teneur en flavonoïdes comprise entre 0,05 et 0,2 mg/ml.

15. Procédé de fabrication d'un médicament à base de plantes selon l'une des revendications 1 à 14, **caractérisé en ce que** l'extrait d'Iberis amara est un extrait de plantes fraîches comportant une teneur en cucurbitacines comprise entre 0 et 200 µg/ml.

16. Procédé de fabrication d'un médicament à base de plantes selon l'une des revendications 1 à 15, **caractérisé en ce que** l'extrait d'Iberis amara est un extrait de plantes fraîches comportant une teneur en cucurbitacines comprise entre 35 et 185 µg/ml.

17. Procédé de fabrication d'un médicament à base de plantes selon l'une des revendications 1 à 16, **caractérisé en ce que** l'extrait d'Iberis amara est un extrait de plantes fraîches comportant une teneur en cucurbitacine I comprise entre 0 et 100 µg/ml et une teneur en cucurbitacine E comprise entre 0 et 100 µg/ml.

18. Procédé de fabrication d'un médicament à base de plantes selon l'une des revendications 1 à 17, **caractérisé en ce que** les extraits de Menthae piperitae folium, Matricariae flos, Carvi fructus, Melissae folium, Angelicae radix, Liquiritiae radix, Cardui mariae fructus et de Chelidonii herba sont des extraits de plantes séchées.

19. Procédé de fabrication d'un médicament à base de plantes selon l'une des revendications 1 à 18, **caractérisé en ce que**, pour les extraits de plantes fraîches, le rapport entre la plante subissant la macération / percolation et l'extrait est compris entre 1 gramme : 10 ml (10 grammes) et 1 gramme : 1 ml (1 gramme).

20. Procédé de fabrication d'un médicament à base de plantes selon l'une des revendications 1 à 19, **caractérisé en ce que**, pour les extraits de plantes fraîches, le rapport entre la plante subissant la macération / percolation et l'extrait est compris entre 1 gramme : 1,5 ml (1,5 grammes) et 1 gramme : 2,5 ml (2,5 grammes).

21. Procédé de fabrication d'un médicament à base de plantes selon l'une des revendications 1 à 20, **caractérisé en ce que**, pour les extraits de plantes séchées, le rapport entre la plante séchée et l'extrait est compris entre 1 gramme : 1 ml (1 grammes) et 1 gramme : 10 ml (10 grammes).

22. Procédé de fabrication d'un médicament à base de plantes selon l'une des revendications 1 à 21, **caractérisé en ce que**, pour les extraits préparés à partir de plantes séchées, le rapport entre les plantes séchées et l'extrait est compris entre 1 gramme : 2 ml (2 grammes) et 1 gramme : 4 ml (4 grammes).

23. Procédé de fabrication d'un médicament à base de plantes selon l'une des revendications 1 à 22, **caractérisé en ce que**, pour les extraits préparés à partir de plantes séchées, le rapport entre les plantes séchées et l'extrait est compris entre 1 gramme : 2,5 ml (2,5 grammes) et 1 gramme : 3,5 ml (3,5 grammes).

24. Procédé de fabrication d'un médicament à base de plantes selon l'une des revendications 1 à 23, **caractérisé en ce que** l'agent d'extraction comprend de l'eau et de l'éthanol aqueux, la concentration en éthanol étant comprise entre 0,1 et 60 % en volume.

25. Procédé de fabrication d'un médicament à base de plantes selon l'une des revendications 1 à 24, **caractérisé en ce que** l'agent d'extraction comprend de l'éthanol aqueux, la concentration en éthanol étant comprise entre 30 et 50 % en volume.

26. Procédé de fabrication d'un médicament à base de plantes selon l'une des revendications 1 à 25, **caractérisé en ce que** l'agent d'extraction destiné à l'extrait de plantes fraîches est de l'éthanol aqueux ayant une concentration en éthanol égale à 50 % en volume, et l'agent d'extraction destiné aux extraits de plantes séchées est de l'éthanol aqueux ayant une concentration en éthanol égale à 30 % en volume.
